# EUROPEAN PATENT SPECIFICATION

(11) **EP 1 558 285 B1**
(45) Date of publication and mention of the grant of the patent: **15.12.2010**
(21) Application number: 03754219.8
(22) Date of filing: 16.10.2003
(51) Int. Cl.: A61K 39/395, A61K 39/00, C07K 14/705

(54) **METHOD FOR DECREASING DEPRESSION BY INHIBITING THE ACTIVITY OF N-TYPE CALCIUM CHANNEL**
VERFAHREN ZUR VERRINGERUNG VON DEPRESSIONEN DURCH HEMMUNG DER WIRKUNG DES KALZIUMKANALS VOM N-TYP
METHODE PERMETTANT DE REDUIRE LA DEPRESSION PAR INHIBITION DE L'ACTIVITE DU CANAL CALCIQUE DE TYPE N

(30) Priority: 17.10.2002 KR 2002063626
(43) Date of publication of application: 03.08.2005
(73) Proprietor: Korea Institute of Science and Technology, Seoul 136-130 (KR)
(72) Inventor: SHIN, Hee-Sup, Jung-ku, 100-754 Seoul (KR); KIM, Chanki, Seongbuk-ku, 136-150 Seoul (KR)
(74) Representative: Grünecker, Kinkeldey, Stockmair & Schwanhäusser Anwaltssozietät
(86) International application number: PCT/KR2003/002159
(87) International publication number: WO 2004/035087

(56) References cited:
- BEUCKMANN CARSTEN T ET AL: "N-type calcium channel alpha1B subunit (Cav2.2) knock-out mice display hyperactivity and vigilance state differences." THE JOURNAL OF NEUROSCIENCE : THE OFFICIAL JOURNAL OF THE SOCIETY FOR NEUROSCIENCE. 30 JUL 2003, vol. 23, no. 17, 30 July 2003 (2003-07-30), pages 6793-6797, XP002368425 ISSN: 1529-2401
- INO MITSUHIRO ET AL: "Functional disorders of the sympathetic nervous system in mice lacking the alpha1B subunit (Cav 2.2) of N-type calcium channels" PROCEEDINGS OF THE NATIONAL ACADEMY OF SCIENCES OF THE UNITED STATES OF AMERICA, vol. 98, no. 9, 24 April 2001 (2001-04-24), pages 5323-5328, XP002368426 ISSN: 0027-8424
- MORI Y ET AL: "Ca<2+> channel [alpha]1B subunit (CaV 2.2) knockout mouse reveals a predominant role of N-type channels in the sympathetic regulation of the circulatory system" TRENDS IN CARDIOVASCULAR MEDICINE 2002 UNITED STATES, vol. 12, no. 6, 2002, pages 270-275, XP002368427 ISSN: 1050-1738
- BATH CATHERINE P ET AL: "The effects of ifenprodil and eliprodil on voltage-dependent Ca-2+ channels and in gerbil global cerebral ischaemia" EUROPEAN JOURNAL OF PHARMACOLOGY, vol. 299, no. 1-3, 1996, pages 103-112, XP002368428 ISSN: 0014-2999
- STOCKER JONATHAN W ET AL: "Preferential interaction of omega-conotoxins with inactivated N-type Ca-2+ channels" JOURNAL OF NEUROSCIENCE, vol. 17, no. 9, 1997, pages 3002-3013, XP002368429 ISSN: 0270-6474
- FENG ZHONG-PING ET AL: "Residue Gly1326 of the N-type calcium channel alpha1B subunit controls reversibility of omega-conotoxin GVIA and MVIIA block" JOURNAL OF BIOLOGICAL CHEMISTRY, vol. 276, no. 19, 11 May 2001 (2001-05-11), pages 15728-15735, XP002368430 ISSN: 0021-9258
- SAEGUSA HIRONAO ET AL: "Suppression of inflammatory and neuropathic pain symptoms in mice lacking the N-type Ca2+ channel" EMBO (EUROPEAN MOLECULAR BIOLOGY ORGANIZATION) JOURNAL, vol. 20, no. 10, 15 May 2001 (2001-05-15), pages 2349-2356, XP002368431 ISSN: 0261-4189
- KIM CHANKI ET AL: "Altered nociceptive response in mice deficient in the alpha1B subunit of the voltage-dependent calcium channel" MOLECULAR AND CELLULAR NEUROSCIENCE, vol. 18, no. 2, August 2001 (2001-08), pages 235-245, XP002368432 ISSN: 1044-7431
- NORMANN C. ET AL.: 'Associative long-term depression in the hippocampus is dependent on postsynaptic N-type Ca2+ channels' J. NEUROSCI. vol. 20, no. 22, 15 November 2000, pages 8290 - 8297, XP008051558
- KING R. D. ET AL.: 'Extracellular calcium depletion as a mechanism of short-term synaptic depression' IN. J. NEUROPHYSIOL. vol. 85, no. 5, 2001, pages 1952 - 1959, XP008051565
- GAO X. B. ET AL.: 'Melanin-concentrating hormone depresses L-, N-, and P/Q-type voltage-dependent calcium channels in rat lateral hypothalamic neurons' J. PHYSIOL. vol. 542, no. 1, July 2002, pages 273 - 286, XP008051564
- MARGRIE T. W. ET AL.: 'Inhibition of transmitter release and long-term depression in the avian hippocampus' NEUROSCI. LETT. vol. 284, no. 1-2, 2000, pages 17 - 20, XP008051560
- AYATA C. ET AL.: 'Impaired neurotransmitter release and elevated threshold for cortical spreading depression in mice with mutations in the alpha1A submit of P/Q type calcium channels' NEUROSCIENCE vol. 95, no. 3, 2000, pages 639 - 645, XP008051575

### SEQUENCE LISTING

<110> KIST
<120> Method for decreasing depression by inhibiting the activity of N-type calcium channel
<130> 3fpo-10-10B
<150> KR2002-63626
   <151> 2002-10-17
<160> 3
<170> KopatentIn 1.71
<210> 1
   <211> 22
   <212> DNA
   <213> Artificial Sequence
<220>
   <223> primer 1
<400> 1
   cagtcaggag ctgtcaagcc ac 22
<210> 2
   <211> 21
   <212> DNA
   <213> Artificial Sequence
<220>
   <223> primer 2
<400> 2
   ccacgaagtc catgacattc c 21
<210> 3
   <211> 21
   <212> DNA
   <213> Artificial Sequence
<220>
   <223> primer 3
<400> 3
   cgggcagctt gccggtggtg c 21

## Description

### FIELD OF THE INVENTION

The present invention relates to *in vitro* screening method for an anti-depression agent.

### BACKGROUND

There are many different kinds of voltage-dependent calcium channels (VDCC) that play important roles in the control of neurotransmitter release, membrane excitability, and gene expression (Catterall, Cell Calcium, 1998, 24, 307-323). These channels have multi-subunit structures consisting of α1, α2, β and γ subunits (Dunlap et al., Trends Neurosci., 1995, 18, 89-98; De Waard et al., Ion Channels, 1996, 4, 41-87; Hofmann et al., Rev. Physiol. Biochem. Pharmacol., 1999, 139, 33-87). VDCCs are classified into various types including L-type, N-type, P-type, Q-type, R-type and T-type according to their electrophysiological or pharmaceutical I characteristics (Tsien et al., Soc. Gen. Physiol. Ser., 1987, 41, 167-187; Randall and Tsien, J. Neusci., 1995, 15, 2995-3012). In general, L-type, N-type, P/Q-type and R-type channels are open with a high voltage, and T-type channel is open with a low voltage. Among voltage-dependent calcium channel subunits, alpha-1 subunit is essential for the channel operation and for the determination of the characteristics of the channel. As of today, 6 different genes (alpha 1A - alpha 1F), encoding in variety of sub-groups of alpha 1 subunit, have been disclosed (Chin., Exp. Mol. Med., 1998, 30, 123-130; Ertel et al., Neuron, 2000, 25, 533-535; Hofmann et al., Rev. Physiol. Pharmacol., 1999, 139, 33-87).

*In situ* hybridization in rat brains has shown that α 1B subunit is widely distributed in various regions of the central nervous system, especially in the dorsal raphe and locus caeruleus regions (TanaKa et al., Brain Res. Mol. Brain Res., 1995, 30, 1-16). At the subcellular level, α 1B subunit is highly localized at the presynaptic nerve terminals of most neurons, consistent with its physiological role in regulating neurotransmitter release (Miller, Science, 1987, 235, 46-52; Hirning et al., Science, 1988, 239, 57-61; Westenbroek et al., J. Neurosci., 1995, 15, 6403-18). In addition, the subunit has also been known to regulate neurotransmission in synapse by being involved in the inhibiting activity of serotonin and norepinephrine in presynapse or in postsynapse (Okada, et al., J. Neurosci., 2001, 21, 628-640; Rittenhous A.R., J. Neurobiol., 1999, 40, 137-148; Sun, Q.Q., et al., J. Physiol., 1998, 510, 103-20).

Recently, results of genetic studies have been reported, which identify the accurate role of N-type calcium channel in handling of nociceptor information *in vivo* by using a mutant mouse deficient in alpha 1B subunit of N-type calcium channel by gene targeting method (Kim et al., Mol. Cellular Neuroscience, 2001, 18, 235-245; Saegusa, H. et al, EMBO J., 2001, 15, 2349-56; Hatakeyama, S. et al, Neuroreport, 2001, 8, 2423-7). According to those studies, the alpha 1B mutant mouse felt a pain much less than a normal mouse, especially for a mechanical pain, a pain by radiant heat and an inflammatory pain. Besides, the mutant mouse was insensitive to a pain accompanied by a harmless stimulation caused by nervous injury and a pain by fever, comparing to a normal mouse. Thus, N-type calcium channel seems to play an important role in recognizing a pain. Another report stated that alpha 1B mutant mouse was confirmed by the elevated plus maze test to feel uneasiness less than a normal mouse (Saegusa, H. et al, EMBO J., 2001, 15, 2349-56). However, pharmacological or genetic studies concerning the effect of N-type calcium channel on emotional troubles such as depression, etc. have not been reported yet.

Thus, the present inventors performed in variety of animal behavior tests relating to depression by using a mutant mouse deficient in N-type calcium channel alpha 1B gene, in order to investigate the relation between depression, a representative emotional trouble, and N-type calcium channel. And the present inventors have completed this invention by confirming that the inhibition of the activity of N-type calcium channel resulted in the reduction of the symptoms of depression.

### SUMMARY OF THE INVENTION

It is an object of this invention to provide an *in vitro* method for screening an anti-depression agent by using an alpha 1B subunit of N-type calcium channel comprising screening of a substance inhibiting the activity of the alpha 1B subunit of the N-type calcium channel, wherein the screening of a substance inhibiting the activity of the alpha 1B subunit of N-type calcium channel comprises the following steps:
1) Obtaining a transformant by transfecting host cells with a vector containing an alpha 1B structural gene and a reporter gene;
2) Culturing the above transformant along with a test sample for screening; and
3) Measuring the expression of the reporter gene.

### DETAILED DESCRIPTION OF PREFERRED EMBODIMENTS

The present invention provides an *in vitro* method for screening an anti-depression drug by using an alpha 1B subunit of N-type calcium channel comprising screening of a substance inhibiting the activity of the alpha 1B subunit of the N-type calcium channel, wherein the screening of a substance inhibiting the activity of the alpha 1B subunit of N-type calcium channel comprises the following steps:
1) Obtaining a transformant by transfecting host cells with a vector containing an alpha 1B structural gene and a reporter gene;
2) Culturing the above transformant along with a test sample for screening; and
3) Measuring the expression of the reporter gene.

Hereinafter, the present invention is described in detail.

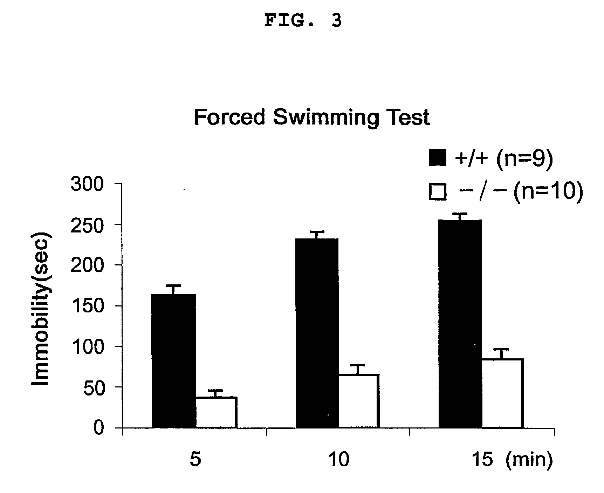

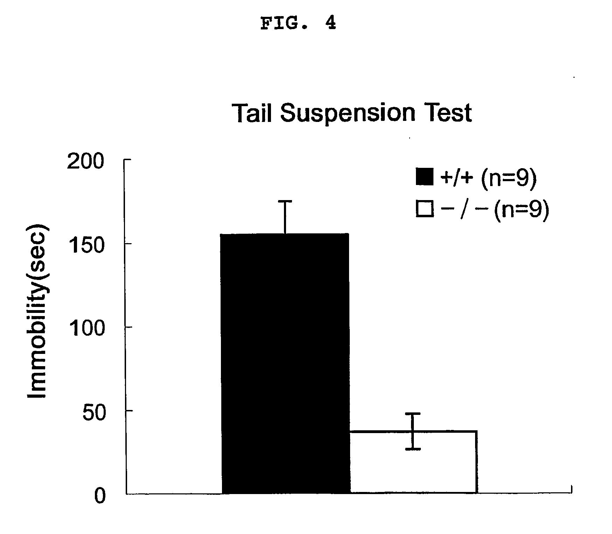

The present inventors investigated the relation between depression and the inhibition of the activity of N-type calcium channel by using a mouse in which N-type calcium channel was not expressed because an alpha 1B gene coding N-type calcium channel was targeted (see FIG. 3 and FIG. 4).

In order to confirm whether N-type calcium channel was related to depression, a representative emotional trouble, the present inventors performed a forced swimming test and a tail suspension test, which are both representative animal behavior tests relating to depression, by using alpha 1B-knockout mice (Korea patent application #2002-2343). First, as for the forced swimming test, both a mutant mouse I and a normal mouse were forced to swim in a beaker for 15 minutes. As a result, while a normal mouse began to show the immobile floating position in 5 minutes, an alpha 1B mutant (knockout) mouse was swimming for 15 minutes all along, suggesting that the immobile floating position time of the mutant mouse was shorter. Thus, it was confirmed that depression in the mutant mouse was remarkably decreased, comparing to that in the normal mouse (see FIG. 3). Second, as for the tail suspension test, the result was similar to that of the forced swimming test, that is, the mutant mouse showed shorter immobilization time than the normal mouse (see FIG. 4). As explained hereinbefore, an alpha 1B-knockout mouse, in which a gene coding N-type calcium channel was targeted, showed an anti-depression response in the two depression related behavior tests above.

It has recently been disclosed that the abnormality of regulation of monoamines such as serotonin and norepinephrine, and substance P, an peptide, is related to depression pathologically and physiologically, so that a substance regulating such neurotransmitters is a prominent candidate for a depression treatment agent (Okada, et al., J. Neurosci., 2001, 21, 628-640; Rittenhous A.R., J. Neurobiol., 1999, 40, 137-148; Sun, Q.Q., et al., J. Physiol., 1998, 510, 103-20). The above alpha 1B-knockout mouse, in which the activity of N-type calcium channel was inhibited, showed the equal behavior response to a normal mouse treated with an anti-depression agent such as selective serotonin reuptake inhibitors (SSRI), tricyclics, monoamine oxidase inhibitors, substance P or NK-1 receptor inhibitor. Serotonin 1A receptor mutant mouse showed much increased anxiety but decreased depression, comparing to a normal mouse. Norepinephrine transporter mutant mouse also showed decreased depression comparing to a normal mouse (Ramboz, S. et al., Proc. Natl. Acad. Sci. USA, 1998, 95, 14476-81; Xu, F. et al., Nature Neuroscience, 2000, 3, 465-471).

Other pharmacological or genetic researches concerning depression or anxiety informed us that depression or anxiety could be decreased by inhibiting substance P which was known to be secreted by N-type calcium channel related to a pain and NK-1 receptor thereof (Santarelli, L. et al., PNAS USA, 2001, 98, 1912-17). Nevertheless, the relation between depression and N-type calcium channel has not been clearly explained yet. Therefore, the present inventors have performed depression related animal behavior tests using a mutant mouse and have clarified for the first time that depression can be decreased by inhibiting the activity of the alpha 1B subunit of N-type calcium channel.

It was also reported from other studies on other calcium channels related to the regulation of an emotion that L-type calcium channel inhibitor `nimodepine' alleviated ultra-rapid-cycling bipolar of bipolar diseases and brief recurrent depression, and a calcium channel alpha 2-delta inhibitors 'gabapentin' and 'lithium' could also be excellent candidates for the treatment agent of such emotional disorder (Post, R.M., et al., Bipolar Disord., 2000, 2, 305-15; Goodnick, P.J., Biopolar Disord., 2000, 2, 165-73). However, as of today, no reports concerning *in vivo* function of a calcium channel related to depression have been made, except the result of the present invention. So, the present inventors have first confirmed the fact that the inhibition of the activity of the alpha 1B subunit of N-type calcium channel is related to decreasing depression.

In conclusion, the inhibition of the activity of N-type calcium channel by suppressing the expression of an alpha 1B protein results in the anti-depression response, suggesting that an N-type calcium channel gene activity inhibitor or its product N-type calcium channel activity inhibitor might be used for the development of a depression treatment agent.

The present invention provides a method for screening an anti-depression agent by using an alpha 1B subunit of N-type calcium channel comprising screening of a substance inhibiting the activity of the alpha 1B subunit of N-type calcium channel, wherein the screening of a substance inhibiting the activity of the alpha 1B subunit of N-type calcium channel comprises the following steps:
1) Obtaining a transformant by transfecting host cells with a vector containing an alpha 1B structural gene and a reporter gene;
2) Culturing the above transformant along with a test sample for screening; and
3) Measuring the expression of the reporter gene.

If ever found, an alpha 1B activity inhibitor can be effectively used as an anti-depression agent. Thus, it is possible to screen a substance inhibiting the activity of an alpha 1B by taking advantage of the general screening method for a substance inhibiting the activity of a protein. The screening of a substance inhibiting the activity of an alpha 1B is composed of the following steps:
1) Obtaining a transformant by transfecting host cells with a vector containing an alpha 1B structural gene and a reporter gene;
2) Culturing the above transformant along with a test sample for screening; and
3) Measuring the expression of the reporter gene.

As a reporter gene in the above, LacZ, GFP and luciferase could be used, but not always limited thereto.

### BRIEF DESCRIPTION OF THE DRAWINGS

The application of the preferred embodiments of the present invention is best understood with reference to the accompanying drawings, wherein:
FIG. 1 is a schematic diagram showing the structures of wild-type alpha 1B locus, targeting vector and disrupted alpha 1B locus,
   A: Wild-type alpha 1B gene locus,
   B: Targeting vector in which IS3 region is replaced with GEP-Neo and tk gene is included,
   C: Gene locus targeted with targeting vector,
   □ : Fragment used as a probe
FIG. 2A is an electrophoresis photograph showing the result of PCR of wild-type, heterozygous, and homozygous mutant mice,
   Lane 1: Marker,
   Lane 2: Wild-type(+/+),
   Lane 3: Heterozygote(+/-),
   Lane 4: Homozygote(-/-)
FIG. 2B is an electrophoresis photograph showing the result of Southern blot analysis of wild-type, heterozygous, and homozygous mutant mice,
   Lane 1 and 2: wild-type(+/+),
   Lane 3 - 5 : Heterozygote(+/-),
   Lane 6 and 7: Homozygote(-/-)
FIG. 2C is an electrophoresis photograph showing the result of Western blot analysis of brain tissues from wild-type and transgenic mice,
   Lane 1: Cerebellum of wild-type mice(+/+),
   Lane 2: Cerebellum of transgenic mice(-/-),
   Lane 3: Cerebrum of wild-type mice(+/+),
   Lane 4: Cerebrum of transgenic mice(-/-)
FIG. 3 is a graph showing the result of forced swimming test performed with wild-type and alpha 1B-knockout mice,
FIG. 4 is a graph showing the result of tail suspension test performed with wild-type and alpha 1B-knockout mice.

### EXAMPLES

Practical and presently preferred embodiments of the present invention are illustrative as shown in the following Examples.

### Example 1: Generation of alpha 1B-knockout mice

### <1-1> Generation of targeting vector deficient in calcium ion channel alpha 1B gene

In order to generate knockout mice for the alpha 1B gene of calcium ion channel, the present inventors had performed gene targeting method. First, bateriophage lamda FIX II library (Stratagene) wherein DNA fragments of 129/sv mouse genome were inserted randomly was screened with alpha 1B rat cDNA (AUG - 720 bp, obtained from Dr. Jin HM, NIH) as a probe to obtain mouse genomic DNA containing alpha 1B gene. Through the screening, bacteriophage clone having 18.4 kb genomic DNA containing the intervening segment (IS) 1-3 coding region of the alpha 1B locus was obtained, which was later used for the generation of a targeting vector after completing a restriction enzyme map using various restriction enzymes. pBluescript II KS(+/-) (Stratagene) was used as an empty vector. From the pEGFP-1 vector (Clontech), green fluorescent protein (GFP) gene containing an SV-40-polyA was cut and fused to the *BamH*| -tagged IS2 containing exon (amino acid 158). The PGK-neo gene originated from pLNT vector (obtained from Sugitaui Noda) was attached to downstream of the GFP.

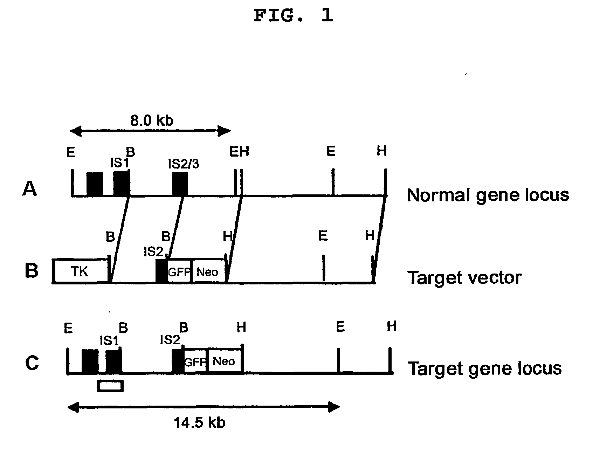

As a result, a targeting vector wherein some parts of IS3 were deleted and replaced with the GFP-NEO cassette for gene disruption and positive selection was constructed. For negative selection, the HSB-tk gene was attached at the 5' end of the 5'-homology region (FIG. 1).

### <1-2> Transfection into embryonic stem cells

J1 embryonic stem cell line was used for the transfection of targeting vector generated in the above Example <1-1>. Embryonic stem cell cultures and embryo manipulations were performed as described in the following reference (Kim et al., Nature, 1997, 389, 290-293). J1 embryonic stem cells (obtained from Dr. R. Jeanisch, MIT, USA) were maintained in DMEM (Gibco Co.) supplemented with 15% fetal bovine serum (Hyclone Co.), 1× penicillin-streptomycin (Gibco Co.), 1×non-essential amino acid (Gibco Co.) at 37°C for 2-3 days. Single cells were obtained by treating the cells with 1 mM EDTA solution containing 0.25% trypsin.

Targeting vector generated in the above Example <1-1> was transfected into the single cells by electroporation. Particularly, 25 µg of targeting vector DNA was added into embryonic stem cells (2×10⁷ cells/Mℓ). After mixing, electroporation was performed with 270 V/500 µF. The cells were cultured in ES medium containing 0.3 mg/Mℓ of G418 and 2 µM of gancyclovior for 5-7 days. Embryonic stem (ES) cell clones correctly targeted were selected by using homologous recombination method, and maintained. After confirming the defection in calcium ion channel alpha 1B gene, the embryonic stem cell clones were distributed into the ES medium again for further culture for 18-22 hours. Single cells were obtained by treating the cells with trypsin. Among them, just live cells were used for microinjection.

### <1-3> Generation of chimera mice

In order to generate chimera mice having alpha 1B +/- genotype, embryonic stem cell clone selected in the above Example <1-2> was microinjected into fertilized blastula. Particularly, female and male C57BL/6J mice (Jackson Laboratory, USA) were mated, and 3.5 days after mating, the female mouse was sacrificed by cervical dislocation. Uterus was removed from the sacrificed female mouse and terminal region of the uterus was cut with scissors. Using 1 Mℓ syringe, 1 Mℓ of injection solution containing 20 mM HEPES, 10% FBS, 0.1 mM 2-mercaptoethanol and DMEM was circulated. Blastula was separated from the above uterus using microglasstube under the dissecting microscopy. The separated blastula was transferred into a drop of the injection solution placed on a 35 mm petridish, which was used for the succeeding introduction process. In order to insert the embryonic stem cell clone selected in the above Example <1-3> into the separated blastula, a microinjection was used (Zeiss Co.). That is, 10-15 embryonic stem cell clones were injected into blastocoel of the blastula. The blastula having the clones was mated with a male which was operated on vasectomy, which was implanted in the uterus of 2.5 p.c. surrogate mother mouse in order to induce the generation of a chimera mouse, a kind of heterozygote formed from the embryonic stem cell clones (J1) and the blastula of C57B/6J mouse. For the implantation, the surrogate mother mouse was anesthetized by avertine (1 mg/kg weight), and the abdomen was cut 1 cm; the upper uterus was taken by a pincette and was pulled outside 2 cm; a hole was made on the uterus by a needle, through which the blastula was injected by using a microglasstube; the inner membrane of abdominal cavity was closed with two stitches and the outside skin was enveloped by a medical clip. The blastula in which the embryonic stem cell clones were inserted was implanted in the uterus of a surrogate mother mouse, followed by incubating for 19 days. So, embryonic stem cell originated cells were fused with blastula originated cells, resulting in the preparation of the chimera mouse having calcium ion channel alpha 1B +/- genotype in its genome.

### <1-4> Generation of alpha 1B +/- heterozygote mice

Among offspring generated from mating a C57BL/6J female mouse with a male chimera mouse obtained in the above Example <1-3>, genetically stable heterozygote F1 transgenic mice were selected. PCR (polymerase chain reaction) was performed to select heterozygote mice having alpha 1B +/- genotype among them.

DNA for PCR was extracted from mice tails. Particularly, 1.5 cm of each mouse tail was cut and dipped in 0.4 Mℓ of lysis buffer containing 100 mM Tris-HCl (pH 8.0), 5 mM EDTA, 200 mM NaCl and 0.2% SDS. Proteinase K (0.1 mg/Mℓ) was added into the above solution and reacted at 55°C for 5 hours. Thereafter, added 75 µℓ 8 M potassium acetate and 0.4 Mℓ chloroform thereto and shook. The solution was suspended at 4°C for 10 minutes. Supernatant and sediment were separated by centrifugation at 15,000 rpm. 1 Mℓ of ethanol was added into the 0.4 Mℓ of separated supernatant to precipitate genomic DNA. The precipitated genomic DNA was washed with 70% ethanol. After drying, the genomic DNA was resolved in 50 µℓ of distilled water and used for PCR.

1 µℓ of the above DNA was used as a template for PCR reaction. 10 pmols of Primer 1 (SEQ. ID. No 1), 2(SEQ. ID. No 2) and 3(SEQ. ID. No 3) were also used as primers for the PCR. A set of primer 1 and primer 2 was designed to amplify 190 bp DNA fragment of wild-type alpha 1B gene and a set of primer 1 and primer 3 was designed to amplify 320 bp DNA fragment of targeted gene. PCR was performed by 40 cycles as follows: a denaturing step at 94°C for 30 seconds, a primer annealing step at 58°C for 30 seconds and an extension step at 72°C for 30 seconds. After PCR, the PCR product was analyzed by 1.5% agarose gel electrophoresis and ethidium bromide (EtBr) staining. For the control, DNA extracted from wild-type mice was used.

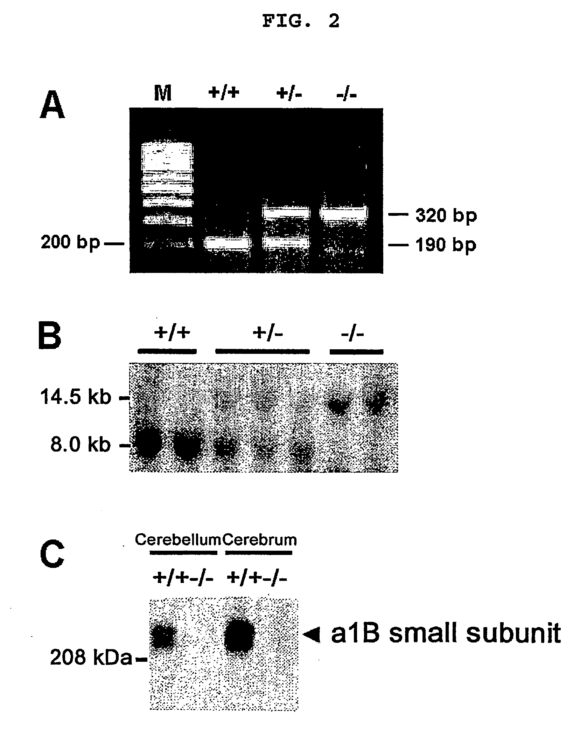

As a result, heterozygote mice having alpha 1B +/- genotype were confirmed by observing that the mice had two different bands; one is a 320 bp size band amplified by targeting alpha 1B gene and the other is a 190 bp size band amplified by wild-type alpha 1B gene (Lane 3, FIG. 2A).

### <1-5> Generation of alpha 1B -/- homozygote mice

Homozygote transgenic mice having alpha 1B -/- genotype were generated by mating a male and a female heterozygote mouse having alpha 1B +/- genotype, which were selected in the above Example <1-4>. Southern blot and PCR were performed to confirm whether the above homozygote transgenic mice had alpha 1B -/- genotype, and Western blot was also performed to confirm that alpha 1B protein was not expressed in the transgenic mice.

First, for the Southern blot analysis, genomic DNA was extracted from transgenic mice tails with the same method as used in the above Example <1-4> and digested with EcoRI. Hybridization was performed using DNA probe obtained from phage DNA of the Example <1-1> (□ region, FIG. 1C) and the above mouse DNA. At this time, genomic DNAs extracted from wild-type and transgenic mouse having alpha 1B +/genotype were used as controls.

As shown in FIG. 2B, a 8.0 kb band originated from normal alpha 1B gene was detected in wild-type (+/+) mouse (Lane 1 and 2), and along with the 8.0 kb band, a 14.5 kb band was also detected in heterozygote (+/-) mouse (Lane 3-5). Meanwhile, only 14.5 kb band originated from an alpha 1B -/- gene was detected in homozygote mouse (Lane 6 and 7), from which it was confirmed that homozygote transgenic mouse had alpha 1B -/- genotype.

Second, after extracting genomic DNA from transgenic mice tails with the same method as used in the above Example <1-4>, PCR was performed, that is; 1 µℓ of genomic DNA was used as a template and the same primers of Example <1-4> were used.

As a result, a 190 bp PCR product amplified from normal alpha 1B gene was detected in wild-type mouse, and along with a 190 bp band, a 320 bp PCR product amplified from alpha 1B +/- gene was detected in heterozygote mouse. Meanwhile, only 320 bp PCR product was detected in homozygote mouse, suggesting that the homozygote transgenic mouse had alpha 1B -/- genotype (FIG. 2A).

Third, the present inventors performed Western blot analysis in order to confirm that alpha 1B gene was not expressed in alpha 1B-knockout mice having alpha 1B -/- genotype. Particularly, wild-type and homogygote transgenic mice were sacrificed by cervical dislocation to isolate cerebrum. The isolated cerebrums were homogenized in cold lysis buffer (50 mM Tris/HCl [pH 7.4], 1 mM EGTA, 1 mM DTT, 1 mM PMSF, complete protease inhibitor cocktail [Boehringer Mannheim], Calpain inhibitors | and ||). After low speed centrifugation (1,000 g, 5 minutes), supernatants were centrifuged (28,000 g, 15 minutes) to obtain crude membrane fractions. The crude membrane fractions were separated in gradient SDS PAGE gels (8%-16%), blotted to nitrocellulose membranes (PROTRAN, Schleicher & Schuell), and visualized by the anti-alpha 1B affinity purified polyclonal antibodies (CW21, Vance et al., J. Biol. Chem., 1998, 273:14495-502) by enhanced chemiluminescence.

As shown in FIG. 2C, an alpha 1B protein was not detected in the alpha 1B -/- brain whereas the protein was detected in the wild type (+/+) mouse brain. Therefore, it was confirmed that calcium ion channel alpha 1B protein was not expressed in alpha 1B-knockout mouse. The present inventors have deposited the embryo of the transgenic mouse having alpha 1B +/- genotype at Korean Collections for Type Cultures of Korea Research Institute of Bioscience and Biotechnology on Jan. 8, 2002 (Accession No: KCTC 10158BP).

### Example 2: Investigation of the effect of N-type calcium channel on depression

In order to observe the response of the alpha 1B-knockout mouse (Accession No: KCTC 10158BP) deficient in N-type calcium channel through depression related animal behavior tests, the present inventors performed a forced swimming test and a tail suspension test. All the test animals could take food and water freely in a breeding place where temperature and humidity were properly controlled and a 12 hour-day/night cycle was given; morning started at 8 o'clock. Both female and male of F1 mice were used for the tests, and their ages were 8-10 week old. All the behavior tests were performed by the rules of a single blind test. All the tests were performed between 9-noon in order to minimize the effect of a 24-hour cycle. And each mouse was used just once for each test.

### <2-1> Reduced immobility in forced swimming test

The forced swimming test was performed according to the method of Posolt et al (Posolt, R.D. et al., Eur. J. Pharmacol., 1978, 47, 379-391) with a slight modification. Particularly, water was filled to 15 cm in a 4ℓ plastic beaker (13 cm × 25 cm), and alpha 1B-knockout mice and control (wild-type) mice were forced to swim therein, causing depression by an inevitable stress. The duration of immobile floating was measured, which reflected the depression-related behavior response. The measurement was made for 15 minutes and data were gathered every 5 minutes. The mean value was used for the analysis.

As a result, while wild-type mice showed immobile floating position from the 5^{th} minute, alpha 1B-knockout mice were swimming all through 15 minutes, showing remarkably short period of immobile floating position, comparing to wild-type mice. From the result, it was confirmed that alpha 1B-knockout mice showed decreased depression response (FIG. 3).

### <2-2> Tail suspension test

The tail suspension test was performed according to the method of Steru et al (Steru, L. et al., Psychopharmacology, 1985, 85, 367-370). Particularly, the tails of mice were taped and suspended for 7 minutes in the middle of a metal rod, making the heads of mice be located at a height of 25 cm from the bottom of a bucket (35 cm × 40 cm). The behavior of each mouse was recorded on videotape and the duration of immobilization time was investigated by a single blind method. A violent behavior was observed for the first 1 minute, which was excluded for the measurement. And for the next 6 minutes, immobilization time expressing the depression was measured. The mean value was used for the analysis.

As a result, like in the above forced swimming test, alpha 1B-knockout mice showed remarkably decreased immobilization time, comparing to wild-type mice in the tail suspension test (FIG. 4). From the result, it was confirmed that alpha 1B-knockout mice showed much decreased depression than wild-type mice.

### INDUSTRIAL APPLICABILITY

As explained hereinbefore, a transgenic mouse deficient in N-type calcium channel showed much decreased depression than a wild-type mouse, suggesting that depression could be decreased by inhibiting the activity of the alpha 1B subunit of N-type calcium channel gene. Therefore, substances inhibiting the activity of alpha 1B subunit N-type calcium channel gene or inhibiting the function of N-type calcium channel, a product of the above gene, can be effectively used as a treatment agent for depression.

## Claims

1. An in vitro screening method for an anti-depression agent by using an alpha 1B subunit of N-type calcium channel comprising screening of a substance inhibiting the activity of the alpha 1B subunit of N-type calcium channel, wherein the screening of a substance inhibiting the activity of the alpha 1B subunit of N-type calcium channel comprises the following steps:
1) obtaining a transformant by transfecting host cells with a vector containing an alpha 1B structural gene and a reporter gene;
2) culturing the above transformant along with a test sample for screening; and
3) measuring the expression of the reporter gene.

## Patentansprüche

1. In-vitro-Screeningverfahren für ein Anti-Depressions-Mittel unter Verwendung einer alpha-1 B-Untereinheit des N-Typ Calciumkanals, umfassend das Screening eines Stoffes, der die Aktivität der alpha-1B-Untereinheit des N-Typ Calciumkanals hemmt, wobei das Screening eines Stoffes, der die Aktivität der alpha-1B-Untereinheit des N-Typ Calciumkanals hemmt, die folgenden Schritte umfasst:
1) Erhalten eines Transformanten durch Transfizieren von Wirtszellen mit einem Vektor, der ein alpha-1 B-Strukturgen und ein Reportergen enthält;
2) Züchten des vorstehenden Transformanten zusammen mit einer Testprobe zum Screening; und
3) Messen der Expression des Reportergens.

## Revendications

1. Procédé de criblage in vitro d'un agent antidépresseur par utilisation d'une sous-unité alpha 1B de canal calcique de type N, comprenant le criblage d'une substance inhibant l'activité de la sous-unité alpha 1B de canal calcique de type N, dans lequel le criblage d'une substance inhibant l'activité de la sous-unité alpha 1B de canal calcique de type N comprend les étapes suivantes :
1) obtention d'un transformant par transfection des cellules hôtes avec un vecteur contenant un gène structurel alpha 1B et un gène rapporteur ;
2) mise en culture dudit transformant en même temps qu'un échantillon de test pour le criblage ; et
3) mesure de l'expression du gène rapporteur.
